# EUROPEAN PATENT SPECIFICATION

(11) **EP 1 774 335 B1**
(45) Date of publication and mention of the grant of the patent: **13.07.2011**
(21) Application number: 05790745.3
(22) Date of filing: 19.07.2005
(51) Int. Cl.: G01N 33/543

(54) **MAGNETIC PARTICLE CAPTURE OF WHOLE INTACT ORGANISMS FROM CLINICAL SAMPLES**
MAGNETPARTIKELERFASSUNG AUS GANZEN INTAKTEN ORGANISMEN KLINISCHER PROBEN
CAPTURE DE PARTICULES MAGNETIQUES D'ORGANISMES INTACTS ENTIERS PROVENANT D'ECHANTILLONS CLINIQUES

(30) Priority: 02.08.2004 US 902871
(43) Date of publication of application: 18.04.2007
(73) Proprietor: Becton, Dickinson and Company, Franklin Lakes, New Jersey 07417-1880 (US)
(72) Inventor: MC MILLAN, Ray, A., Timonium. MD 21093 (US)
(74) Representative: von Kreisler Selting Werner
(86) International application number: PCT/US2005/025511
(87) International publication number: WO 2006/020280

(56) References cited:
- EP-A- 1 312 671
- DAUER, R. R. AND DUNLOP, E.H.: "High gradient magnetic separation of yeast" BIOTECHNOLOGY AND BIOENGINEERING, vol. 37, 1991, pages 1021-1028, XP009058580
- OLSEN E ET AL: "Screening for Campylobacter directly in chicken faeces using paramagnetic particles with general affinity for pathogenic bacteria, followed by PCR amplification" ABSTRACTS OF THE GENERAL MEETING OF THE AMERICAN SOCIETY FOR MICROBIOLOGY, vol. 102, 2002, page 445, XP009058576 & 102ND GENERAL MEETING OF THE AMERICAN SOCIETY FOR MICROBIOLOGY; SALT LAKE CITY, UT, USA; MAY 19-23, 2002 ISSN: 1060-2011

## Description

### Technical Field

The present invention is directed to compositions and methods for extracting, concentrating and/or isolating whole, intact particles or organisms from a sample. More particularly, the present invention is directed to compositions and methods for extracting, concentrating and/or isolating whole, intact particles or organisms from samples via reversible binding with magnetically-responsive particles.

### Background Art

In the following discussion certain articles and methods will be described for background and introductory purposes. Nothing contained herein is to be construed as an "admission" of prior art. Applicant expressly reserves the right to demonstrate, where appropriate, that the articles and methods referenced herein do not constitute prior art under the applicable statutory provisions.

The isolation and/or separation of biological components from a sample is a necessary task in many diagnostic and biochemical procedures. Known techniques for accomplishing this objective include lysing of biological materials to release the nucleic acids contained therein, followed by separation of at least a portion of the nucleic acid. The nucleic acid can be separated and/or removed via a number of different techniques. One such technique involves reversibly binding the nucleic acid to magnetic particles. Such techniques are described in U.S. Patent Nos. 5,973,138 and 6,433,160. It is desirable, however, to concentrate, isolate or remove whole, intact particles or organisms from a sample.

### Disclosure of Invention

The present invention is directed to compositions and techniques that non-specifically associate whole, intact particles or organisms with magnetic particles by altering the surface charge characteristics of the magnetic particles and/or the surface charge characteristics of the particles or organisms themselves. Thus, the whole, intact particles or organisms are non-specifically associated with the magnetic particles without precipitation of these particles or organisms out of solution.

According to one aspect, the present invention provides a method comprising providing a sample containing at least one whole, intact particle or organism, creating a mixture, that comprises the sample, at least one magnetically-responsive particle, and a remainder, and providing the mixture with a pH of less than about 7.0. By providing the mixture with a pH of less than about 7.0, alteration of surface charge properties of at least the one magnetically-responsive particle occurs, thereby causing the at least one whole, intact particle or organism to become non-specifically bound to the at least one magnetically-responsive particle to form a complex.

### Brief Description of the Drawings

The foregoing and other features, aspects and advantages of the present invention will become apparent from the following description, appended claims and the exemplary embodiments shown in the drawing, which is briefly described below. It should be noted that, unless otherwise specified, like elements have the same reference numbers.

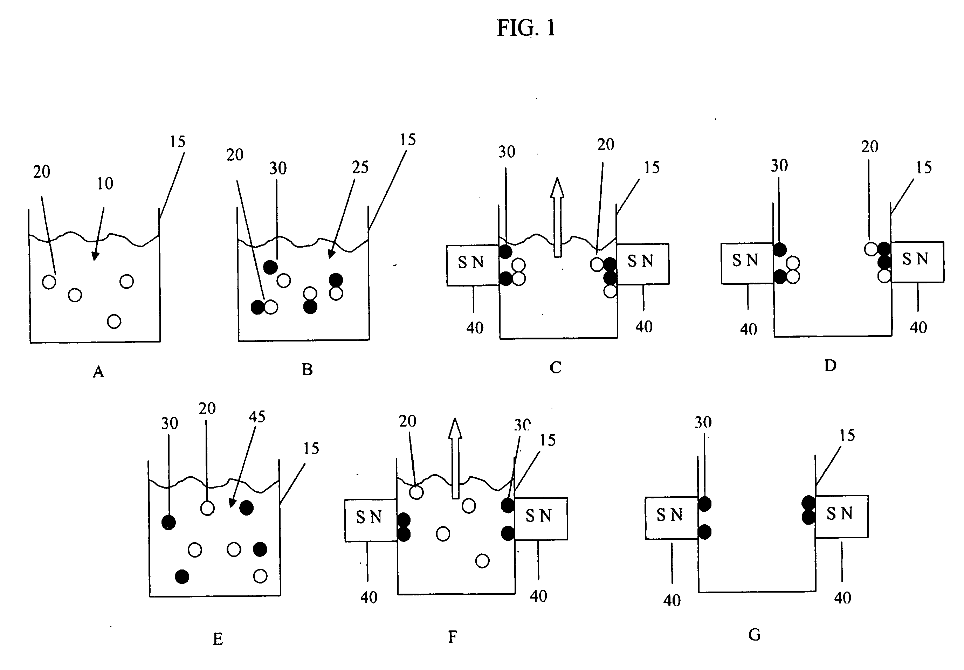

FIG. 1 is a schematic illustration of an embodiment of a process performed according to the principles of the present invention.

### Modes for Carrying Out The Invention

The principles of the present invention will now be further described by the following discussion of certain illustrative embodiments thereof and by reference to the foregoing drawing figure.

As used herein, "whole, intact particles or organism" means any naturally occurring or synthetic modification of a whole particle or organism that has not been lysed or otherwise broken down into constituent components. Whole, intact particles or organisms include, but are not limited to, whole cells, bacteria, viruses, parasites and combinations of the foregoing.

As used herein, "sample" means any biological particle or organism-containing substance including, but not limited to, blood, plasma, serum, urine, bone marrow aspirates, cerebral spinal fluid, tissue, cells, food, feces, saliva, oral secretions, nasal secretions, bronchial lavage, cervical fluids and lymphatic fluids. Optionally, the sample may be sterile.

As used herein, "magnetically-responsive particle" means a particle is capable of having a magnetic moment imparted thereto or otherwise moveable under the action of a magnetic field.

As used herein, "non-specifically bound" means the binding mechanism does not occur via a receptor, capture agent, or the like, which would selectively couple with a specific agent.

The Applicant has found that when in an acidic environment, magnetically-responsive particles will reversibly bind to whole, intact particles or organisms. Although not desiring to be bound by a particular theory, the Applicant believes that an acidic environment increases the electropositive nature of the particles, thereby increasing the binding of the particles to the electronegative whole, intact particles or organisms.

According to a preferred embodiment of the present invention, the magnetically-responsive particles are preferably uncoated or otherwise untreated. Thus, the particles bind non-specifically to the whole, intact particles or organisms. Particles useful in the present invention include iron particles, and the iron may be an iron oxide of forms such as ferric hydroxide and ferrosoferric oxide, which have low solubility in an aqueous environment. Other iron particles such as iron sulfide and iron chloride may also be suitable for binding and extracting nucleic acids using the conditions described herein.

The shape of the magnetically-responsive particles is not critical to the present invention. The magnetically-responsive particles may be of various shapes including, for example, spheres, cubes, oval, capsule-shaped, tablet-shaped, nondescript random shapes, etc., and may be of uniform shape or non-uniform shapes. Whatever the shape of a particle, its diameter at its widest point is generally in the range of from about 0.1µm to about 20µm. According to one embodiment, the magnetically-responsive particles have a diameter of about 1.0µm.

The acidic environment in which the magnetically-responsive particles effectively and reversibly bind whole, intact particles or organisms can be provided through a variety of means. For example, the magnetically-responsive particles can be added to an acidic solution, or an acidic solution may be added to the particles. Alternatively, a solution or environment in which the magnetically-responsive particles are located can be acidified by addition of an acidifying agent such as hydrochloric acid, sulfuric acid, acetic acid or citric acid. Provided that the environment in which the magnetically-responsive particles are located is of a pH less than about 7.0, the particles will reversibly bind whole, intact particles or organisms. According to a preferred embodiment, a pH of about 4.5-5.5 is established to promote binding.

One or more washing steps may optionally be performed at this stage to further eliminate undesirable substances. Any suitable wash may be utilized. For example, a non-ionic detergent or a non-ionic detergent/low concentration acid solution may be utilized.

The bound whole, intact particles or organisms can be eluted into an appropriate buffer for further manipulation. Heating the.environment of the particles with bound whole, intact particles or organisms and/or raising the pH of such environment can accomplish such elution. Agents that can be used to aid the elution of whole, intact particles or organisms from magnetically-responsive particles include basic solutions such as potassium hydroxide, sodium hydroxide or any compound that will increase the pH of the environment to an extent sufficient that electronegative whole, intact particles or organisms are displaced from the magnetically-responsive particles. According to a preferred embodiment, a pH of about 8.3-8.4 is established to promote release of the bound particles or organisms.

The whole, intact particles or organisms can then be extracted, concentrated and/or isolated. Subsequently, the whole, intact particles or organisms can be subjected to further processes, such as one or more of the following: cultivation, polymerase chain amplification, strand displacement amplification, reverse transcriptase strand displacement amplification, and ligase chain amplification.

An exemplary process performed according to the principles of the present invention will now be described by reference to FIG. 1.

In step A, a sample 10 is located in a container 15. The sample 10 contains whole, intact particles or organisms 20.

A mixture 25 is then formed in step B that includes the sample 10, whole, intact particles or organisms 20 and magnetically-responsive particles 30. The pH of this mixture is brought to an appropriate level, preferably below about 7.0, more preferably about 4.5-5.5. The mixture can be formed by any suitable means. For example, the magnetically-responsive particles 30 can be added to an acidic solution, or an acidic solution may be added to the particles 30. Alternatively, a solution or environment in which the magnetically-responsive particles 30 are located can be acidified by addition of an acidifying agent such as hydrochloric acid, sulfuric acid, acetic acid or citric acid.

As previously described, the change in pH causes a modification of the surface charge characteristics of at least the magnetically-responsive particles 30, causing the whole, intact particles or organisms 20 to become bound to the magnetically-responsive particles 30, thereby forming a complex. A magnetic field is then applied. As illustrated in step C, this can be accomplished by bringing opposing permanent magnets 40, or electromagnets (not shown), into close proximity with the outside of the container 15. Under the influence of the magnetic field, the bound whole, intact particle or organism magnetically-responsive particle complex is drawn toward the magnets. The supernatant, or remainder, of the mixture 25 can them be removed from the container 15 (step D).

One or more washing steps (not shown) may optionally be performed at this stage to further eliminate undesirable substances. Any suitable wash may be utilized. For example, a non-ionic detergent or a non-ionic detergent/low concentration acid solution may be utilized.

Step E is illustrative of eluting the complex to free the whole intact particles or organisms 20 from the magnetically-responsive particles 30. This elution can be accomplished by any suitable means such as by chemical agent, thermal energy or a combination of the two. For example, a buffer agent 45 can be added to increase the pH to a suitable level. According to one embodiment, the pH is raised to approximately 8.3-8.4. The buffer may comprise KOH.

The magnets 40 are then brought back into close proximity with the container 15 in step F, which now draws just the magnetically-responsive particles 30 to the sidewalls of the container 15. The whole, intact particles or organisms 20 can then be removed from the container 15 (step G).

Subsequent to step G, the whole, intact particles or organisms 20 can be subjected to further processes, such as one or more of the following: cultivation, polymerase chain amplification, strand displacement amplification, reverse transcriptase strand displacement amplification, and ligase chain amplification.

The above-described steps of the exemplary process may be carried out manually, in automated fashion or by a combination of manual and automated steps. The automated steps may be performed with an automated robotic device, which optionally includes automated pipetting, mixing, and magnet positioning functionality. The automated robotic device may be computer controlled.

The present invention can be used in a number of different contexts. For example, the present invention may be utilized in connection with systems and methods of the type described in U.S. Patent No. 6,672,458.

The principles if the present invention will now be describe by reference to the following illustrative, non-limiting examples.

### Example 1

An experiment was performed to determine whether Staphylococcus aureus (S. aureus) and Escherichia coli (E. coli) could be extracted from an acidic buffer environment. The recovery of the microorganisms from the buffer was evaluated by examination of cultures prepared as described below.

A 1.0 ml quantity of 0.1M sodium acetate buffer having a pH of 4.8 was pipetted into 2.0ml microcentrifuge tubes, each tube containing 50mg of ferrosferric oxide having an average particle size of approximately 1.4 microns. A 0.01ml quantity of a S. aureas ATCC 25923 suspension at 1 x 106 CFU/ml was added to one tube, and a 0.01ml quantity of E. coli ATCC 11775 suspension at 1 x 106 CFU/ml was added to a second tube.

The tubes containing the above-described mixture were rotated on a Nutator mixer for three hours at ambient temperature to promote binding of the iron oxide with the S. aureus and E. coli microorganisms. A neodymium magnet was then placed at the sides of the tubes for 30 seconds.

The supernatant was then removed from the tubes with a pipette. Some of the removed supernatant was used to make a 10-fold dilution. Both the undiluted and the diluted supernatant were applied to growth plates as described in more detail below.

The iron oxide/microorganism complex in the microtube was then washed twice with the above-mentioned sodium acetate buffer. The complex was then resuspended with 1ml of the sodium acetate buffer. A portion of the suspension was then used to prepare a 10-fold dilution. Both the undiluted and the diluted suspension were applied to growth plates as described in more detail below.

A 0.1 ml quantity of each of the following samples were pipetted and spread onto each one of 3 different BBLTM blood agar plates (TSA II with 5% sheep's blood):
(i) undiluted S. aureus supernatant;
(ii) diluted S. aureus supernatant;
(iii) undiluted S. aureus iron oxide suspension;
(iv) diluted S. aureus iron oxide suspension;
(v) undiluted E. coli supernatant;
(vi) diluted E.coli supernatant;
(vii) undiluted E. coli iron oxide suspension; and
(viii) diluted E. coli iron oxide suspension.

The plates were incubated at 36°C in ambient air for 24 hours. To determine the total recovery, the number of colonies were counted on each plate and multiplied by 10 for the undiluted sample, and multiplied by 100 for the diluted sample. The number of colonies calculated are reported in Tables I and II below.

**Table I - S. aureus recovery**

| Sample | Plate 1 | Plate 2 | Plate 3 | Average |
|---|---|---|---|---|
| (i) | 0 | 0 | 0 | 0 |
| (ii) | 0 | 0 | 0 | 0 |
| (iii) | TNTC* | TNTC | TNTC | TNTC |
| (iv) | 23400 | 16800 | 19900 | 20033 |

| | | | | |
|---|---|---|---|---|
| * = Too Numerous To Count (TNTC) | | | | |

**Table II - E. coli recovery**

| Sample | Plate 1 | Plate 2 | Plate 3 | Average |
|---|---|---|---|---|
| (v) | 40 | 60 | 80 | 60 |
| (vi) | 0 | 0 | 100 | 33 |
| (vii) | 1980 | 2730 | 710 | 1807 |
| (viii) | 2000 | 600 | 400 | 1000 |

From the above-reported data, it is evident that both S. aureus and E. coli were captured via binding to the iron oxide in the sodium acetate buffer at pH 4.8. By contrast, a significantly smaller number of microorganisms appear to be in the supernatant (i.e., unbound to the iron oxide).

### Example 2

An experiment was performed to determine whether Staphylococcus aureus (S. aureus) could be extracted from a pooled urine sample. The recovery of the microorganism from the urine was evaluated by examination of cultures prepared as described below.

The pH of pooled urine from healthy male and female donors was adjusted to pH 4.8 with 0.1M acetate buffer having a pH of 4.8. A 1.0ml quantity of pH-adjusted urine was pipetted into a 2.0ml microcentrifuge tube containing 50mg of ferrosferric oxide having an average particle size of approximately 1.4 microns. A 0.01ml quantity of a S. aureas ATCC 25923 suspension at 1 x 106 CFU/ml was added to the tube.

The tube containing the above-described mixture were rotated on a Nutator mixer for two hours at ambient temperature to promote binding of the iron oxide with the S. aureus microorganisms. A neodymium magnet was then placed at the sides of the tubes for 30 seconds.

The supernatant was then removed from the tubes with a pipette. The undiluted supernatant was applied to growth plates as described in more detail below.

The iron oxide/microorganism complex in the microtube was then washed twice with the above-mentioned sodium acetate buffer. The complex was then resuspended with 1ml of 0.154M sodium chloride solution. The undiluted suspension was applied to growth plates as described in more detail below.

A 0.1ml quantity of each of the above-mentioned supernatant and suspension were pipetted and spread onto each one of 3 different BBLTM blood agar plates (TSA II with 5% sheep's blood). The plates were incubated at 36°C in ambient air for 24 hours. To determine the total recovery, the number of colonies were counted on each plate and multiplied by 10. The numbers of colonies calculated are reported in Table III.

**Table III - S. aureus recovery**

| Sample | Plate 1 | Plate 2 | Plate 3 | Average |
|---|---|---|---|---|
| Supernatant | 3440 | 4290 | 3630 | 3786 |
| Suspension > | 10,000* | > 10,000 | > 10,000 | > 10,000 |

| | | | | |
|---|---|---|---|---|
| * = Too numerous to count entire plate, so ¼ of one plate counted, multiplied by 4, then by 10 to arrive at rough estimate for all plates. | | | | |

From the above-reported data, it is evident that both S. aureus was captured via binding to the iron oxide in the sodium acetate buffer at pH 4.8. By contrast, a significantly smaller number of microorganisms appear to be in the supernatant (i.e., unbound to the iron oxide).

While this invention is satisfied by embodiments in many different forms, as described in detail in connection with preferred embodiments of the invention, it is understood that the present disclosure is to be considered as exemplary of the principles of the invention and is not intended to limit the invention to the specific embodiments illustrated and described herein. Numerous variations may be made by persons skilled in the art without departure from the spirit of the invention. The scope of the invention will be measured by the appended claims and their equivalents.

The foregoing presentation of the described embodiments is provided to enable any person skilled in the art to make or use the present invention. Various modifications to these embodiments are possible, and the generic principles presented herein may be applied to other embodiments as well. The abstract is not to be construed as limiting the scope of the present invention, as its purpose is to enable the appropriate authorities, as well as the general public, to quickly determine the general nature of the invention.

## Claims

1. A method comprising:
(i) providing a sample containing at least one whole, intact particle or organism in a container;
(ii) creating a mixture comprising the sample, at least one magnetically-responsive, uncoated, untreated particle, and a remainder; and
(iii) providing the mixture with a pH of less than 7.0,
wherein alteration of the surface charge properties of at least the one magnetically-responsive, uncoated, untreated particle occurs, thereby causing the at least one whole, intact particle or organism to become non-specifically bound to the at least one magnetically-responsive, uncoated, untreated particle to form a complex, wherein the binding of the at least one whole, intact particle or organism is reversible;
(iv) applying a magnetic field to the complex;
(v) removing the remainder of the mixture from the container while the magnetic field is applied to the complex;
(vi) washing the complex; and
(vii) eluting the at least one whole, intact particle or organism from the at least one magnetically-responsive, uncoated, untreated particle comprising raising the pH to 8.3-8.4.

2. The method of claim 1, further comprising:
(viii) reapplying a magnetic field to the eluted at least one magnetically-responsive, uncoated, untreated particle thereby separating the at least one magnetically-responsive, uncoated, untreated particle from the at least one whole, intact particle or organism.

3. The method of claim 2, further comprising:
(ix) removing the at least one whole, intact particle or organism from the container.

4. The method of claim 1, wherein the magnetically-responsive, uncoated, untreated particle comprises iron oxide, ferric hydroxide or ferrosoferric oxide.

5. The method of claim 1, wherein step (iii) comprises providing the mixture with a pH of 4.5-5.5.

## Patentansprüche

1. Verfahren umfassend:
(i) Bereitstellen einer Probe, die wenigstens ein ganzes intaktes Teilchen oder einen ganzen intakten Organismus enthält, in einem Behälter;
(ii) Erzeugen eines Gemischs, das die Probe, wenigstens ein magnetisches, unbeschichtetes, unbehandeltes Teilchen und einen Rest umfasst; und
(iii) Versehen des Gemischs mit einem pH von weniger als 7,0;
wobei eine Veränderung der Oberflächenladungseigenschaften wenigstens des einen magnetischen, unbeschichteten, unbehandelten Teilchens erfolgt, wodurch bewirkt wird, dass das wenigstens eine ganze intakte Teilchen oder der ganze intakte Organismus unspezifisch an das wenigstens eine magnetische, unbeschichtete, unbehandelte Teilchen bindet, wobei ein Komplex entsteht, wobei die Bindung des wenigstens einen ganzen intakten Teilchens oder der ganze intakte Organismus reversibel ist;
(iv) Anlegen eines Magnetfelds an den Komplex;
(v) Entfernen des Rests des Gemischs aus dem Behälter, während das Magnetfeld an den Komplex angelegt ist;
(vi) Waschen des Komplexes; und
(vii) Eluieren des wenigstens einen ganzen intakten Teilchens oder Organismus von dem wenigstens einen magnetischen, unbeschichteten, unbehandelten Teilchen durch Erhöhen des pH-Werts auf 8,3 bis 8,4.

2. Verfahren gemäß Anspruch 1, weiterhin umfassend:
(viii) erneutes Anlegen eines Magnetfelds an das eluierte, wenigstens eine magnetische, unbeschichtete, unbehandelte Teilchen, wodurch das wenigstens eine magnetische, unbeschichtete, unbehandelte Teilchen von dem wenigstens einen ganzen intakten Teilchen oder Organismus getrennt wird.

3. Verfahren gemäß Anspruch 2, weiterhin umfassend:
(ix) Entfernen des wenigstens einen ganzen intakten Teilchens oder Organismus aus dem Behälter.

4. Verfahren gemäß Anspruch 1, wobei das magnetische, unbeschichtete, unbehandelte Teilchen Eisenoxid, Eisen(III)hydroxid oder Eisen(II,III)oxid umfasst.

5. Verfahren gemäß Anspruch 1, wobei Schritt (iii) das Versehen des Gemischs mit einem pH von 4,5 bis 5,5 umfasst.

## Revendications

1. Procédé comprenant :
(i) la fourniture d'un échantillon contenant au moins une particule ou un organisme entières intacte dans un récipient ;
(ii) la création d'un mélange comprenant l'échantillon, au moins une particule non traitée, non enrobée, réactive d'un point de vue magnétique et un reste ; et
(iii) la fourniture du mélange avec un pH inférieur à 7,0,
où la modification des propriétés de charge de la surface de la au moins une particule non traitée, non enrobée, réactive d'un point de vue magnétique se produit, faisant de cette manière que la/le au moins un(e) particule ou organisme entière/entier intact(e) se retrouve lié(e) de manière non spécifique à la au moins une particule non traitée, non enrobée, réactive d'un point de vue magnétique pour former un complexe, où la liaison de la/du au moins un(e) particule ou organisme entière/entier intact(e) est réversible ;
(iv) l'application d'un champ magnétique sur le complexe ;
(v) l'élimination du reste du mélange à partir du récipient alors que le champ magnétique est appliqué sur le complexe ;
(vi) le lavage du complexe ; et
(vii) l'élution de la/du au moins un(e) particule ou organisme entière/ entier intact(e) à partir de la au moins une particule non traitée, non enrobée, réactive d'un point de vue magnétique comprenant l'élévation du pH à 8,3-8,4.

2. Procédé selon la revendication 1, comprenant en outre :
(viii) la réapplication d'un champ magnétique sur la au moins une particule non traitée, non enrobée, réactive d'un point de vue magnétique éluée, séparant de cette manière la au moins une particule non traitée, non enrobée, réactive d'un point de vue magnétique de la/du au moins un(e) particule ou organisme entière/entier intact(e).

3. Procédé selon la revendication 2, comprenant en outre :
(ix) le prélèvement de la/du au moins un(e) particule ou organisme entière/entier intact(e) à partir du récipient.

4. Procédé selon la revendication 1, dans lequel la particule non traitée, non enrobée, réactive d'un point de vue magnétique comprend de l'oxyde de fer, de l'hydroxyde ferrique ou de l'oxyde ferrosoferrique.

5. Procédé selon la revendication 1, dans lequel l'étape (iii) comprend la fourniture du mélange avec un pH de 4,5 à 5,5.
